# EUROPÄISCHE PATENTANMELDUNG

(11) **EP 1 209 459 A1**
(43) Veröffentlichungstag der Anmeldung: **29.05.2002**
(21) Anmeldenummer: 01113910.2
(22) Anmeldetag: 07.06.2001
(51) Int. Cl.: G01N 1/14, G01N 33/18

(54) **Probenehmer und Verfahren zum Abfüllen und Abkühlen eines Fluids**

(30) Priorität: 24.11.2000 EP 00125777
(71) Anmelder: ENDRESS + HAUSER WETZER GmbH + Co. KG, 87481 Nesselwang (DE)
(72) Erfinder: Zeller, Robert, 87439 Kempten (DE)
(74) Vertreter: Andres, Angelika

(57) **Zusammenfassung**

Der Probenehmer umfaßt eine Gefäßanordnung (2) zum Entnehmen, Führen und Speichern einer bei einer vorgebbaren Lagerungs-Temperatur einzulagernden Fluid-Probe sowie eine mit der Gefäßanordnung (2) thermisch gekoppelte Kühlanordnung (3) zum Abkühlen der Fluid-Probe. Die Kühlanordnung (3) weist ein die Gefäßanordnung (2) umhüllendes erstes Kühlvolumen und ein die Gefäßanordnung (2) ummantelndes zweites Kühlvolumen auf. Zum Abkühlen der Fluid-Probe wird eine Gefäßanordnungs-Innentemperatur mittels der Kühlanordnung (3) vor und/oder während des Abfüllens der Fluid-Probe abgesenkt und das Fluid anschließend in der Gefäßanordnung (2) geführt bzw. gehalten.

## Beschreibung

Die Erfindung betrifft einen Probenehmer und ein Verfahren zum Abfüllen und Abkühlen eines Fluids.

Fluide unterschiedlichster Herkunft und Anwendung sind häufig auf ihren chemisch-biologischen Zustand hin zu überwachen, der durch im Fluid mitgeführte Substanzen mitbestimmt wird. Zur Überwachung von wäßrigen Fluiden, insb. in der Trinkwasseraufbereitung oder in der Abwasserreinigung, sind dazu an entsprechenden Fluid-Entnahmestellen räumlich und zeitlich verteilt, repräsentative Fluid-Proben zu entnehmen und entsprechend zu untersuchen. Das Volumen derartiger Fluid-Proben liegt üblicherweise zwischen ca. 10 ml und 500 ml.

Die Entnahme und das Abfüllen erfolgt üblicherweise mittels eines Probenehmers. So ist in der US-A 37 95 347, US-A 38 80 011, US-A 44 15 011, US-A 55 87 926 z.B. ein Probenehmer zum Abfüllen einer Fluid-Probe eines an einer Fluid-Entnahmestelle entnommenen Fluids beschrieben, der umfaßt:
- eine Gefäßanordnung
   -- mit einem rohrförmigen Entnahmegefäß zum Führen eines strömenden Fluids und
   -- mit einem Speichergefäß zum Speichern der Fluid-Probe.

Außerdem weisen Probenehmer der beschriebenen Art üblicherweise eine entsprechende, insb. von einer Steuer-Elektronik gesteuerte, Pumpvorrichtung auf, mittels der ein Einströmen des Fluids in die Gefäßanordnung bewirkt wird. Als Pumpvorrichtung wird oftmals, wie z.B. in der US-A 38 80 01.1, US-A 40 77 263, US-A 46 60 607 oder US-A 55 87 926 beschrieben eine Vakuumpumpe bzw. eine Verdrängerpume, insb. eine mechanisch auf das Entnahmegefäß einwirkende Peristaltik-Pumpe, verwendet.

Ferner kann die Gefäßanordnung des Probenehmers, wie z.B. in der US-A 38 97 687 oder der US-A 37 95 347 beschrieben, ein Dosiergefäß zum Abdosieren der Fluid-Probe vom entnommen Fluid aufweisen.

Üblicherweise umfaßt der Probenehmer des weiteren ein einziges Probenehmer-Gehäuse, in dem die Gefäßanordnung, die Pumpvorrichtung sowie elektronische Baueinheiten untergebracht sein können.

Innerhalb des Fluid laufen aufgrund der mitgeführten Substanzen, insb. aufgrund von Bakterien, aber auch aufgrund bestimmter chemischer Verbindungen, Stoffwechselvorgänge ab, die den chemisch-biologischen Zustand des Fluids praktisch ständig verändern. Diese Änderung des chemisch-biologischen Zustand des Fluids je Zeiteinheit wird üblicherweise als Aktivität des Fluids bezeichnet. Die Aktivität ist temperaturabhängig und praktisch umso größer, je höher die Temperatur des Fluids, insb. auch der Fluid-Probe, ist.

Die Güte der Überwachung wird u.a. dadurch bestimmt, wie gut der chemisch-biologische Zustand der Fluid-Probe zum Zeitpunkt der Untersuchung mit dem chemisch-biologischen Zustand des Fluids zum Zeitpunkt der Entnahme übereinstimmt. Entnahme und Untersuchung der Fluid-Probe finden jedoch häufig über einen längeren Zeitraum voneinander getrennt statt. Daher werden die Fluid-Proben in geeignete Speichergefäße, z.B. Probeflaschen, abdosiert und in diesen eingelagert.

Soll die Fluid-Probe bei der Untersuchung den zum Zeitpunkt der Entnahme im Fluid vorhandenen chemisch-biologischen Zustand möglichst genau repräsentieren, so muß die über den Zeitraum zwischen Entnahme und Untersuchung gemittelte Aktivität der Fluid-Probe entsprechend minimiert werden. Üblicherweise werden die Fluid-Proben daher, wie z.B in der US-A 55 87 926 oder der WO-A 90 14 586 gezeigt, auf eine stationäre Lagerungs-Temperatur, von z.B. 4 °C (= 277 K), abgekühlt, bei der eine zulässige Aktivität nicht überschritten wird.

Zum Abkühlen der Fluid-Proben werden die Speichergefäße in einem Kühlraum, der wie z.B. in der US-A 55 87 926 vorgeschlagen, direkt im Probenehmer-Gehäuse angeordnet sein kann, bei einer entsprechenden Lagerungs-Temperatur untergebracht und die Fluid-Proben mittels des Probenehmers von der Fluid-Entnahmestelle praktisch direkt in die Speichergefäße abgefüllt und dort abgekühlt.

Es hat sich jedoch gezeigt, daß bei diesem Verfahren die Abkühlgeschwindigkeiten, insb. bei einer Fluid-Temperatur an der Fluid-Entnahmestelle von über 15 °C, zu gering und damit die Aktivität der einzulagernden Fluid-Probe bis zur Untersuchung zu groß sein können. So wurden bei einer Fluid-Temperatur von 16 °C und einem Volumen der Fluid-Probe von 500 ml eine mittlere Abkühlgeschwindigkeit von z.B. 1 K/h und damit eine Abkühlzeit bis zum Erreichen der Lagerungs-Temperatur von 12 h gemessen. Aufgrund der zu großen Aktivität der Fluid-Probe kann sich der chemisch-biologische Zustand der Fluid-Probe während des Abfüllens und Abkühlens erheblich im Vergleich zum momentanen chemisch-biologischen Zustand bei der Entnahme verändern.

Eine Aufgabe der Erfindung besteht darin, einen Probenehmer zum Abfüllen und Abkühlen einer solchen Fluid-Probe anzugeben, mit dem die Abkühlzeit und damit die Aktivität der Fluid-Probe reduziert werden kann.

Eine weitere Aufgabe der Erfindung ist es, ein entsprechendes Verfahren zum Abfüllen und Abkühlen einer derartigen Fluid-Probe anzugeben, dessen Anwendung eine Reduzierung der Abkühlzeit bewirkt.

Zur Lösung der Aufgabe besteht die Erfindung in einem Probenehmer zum Abfüllen und Abkühlen einer bei einer vorgebbaren Lagerungs-Temperatur einzulagernden Fluid-Probe eines an einer Fluid-Entnahmestelle entnommenen Fluids, das eine momentane Entnahme-Temperatur aufweist, die größer als die Lagerungs-Temperatur ist, welcher Probenehmer umfaßt:
- eine Gefäßanordnung von vorgebbarem Lumen mit
   -- einer über das Lumen räumlich gemittelten Gefäßanordnungs-Innentemperatur
   -- einem rohrförmigen Entnahmegefäß zum Führen des entnommenen Fluids und
   -- einem Speichergefäß zum Speichern der Fluid-Probe sowie
- eine mit der Gefäßanordnung thermisch gekoppelte Kühlanordnung zum Einstellen der Gefäßanordnungs-Innentemperatur, mit
   -- einem wenigstens das Speichergefäß umhüllenden ersten Kühlvolumen von vorgebbarer erster Kühltemperatur zum Kühlen der Fluid-Probe auf die Lagerungs-Temperatur und
   -- einem die Gefäßanordnung wenigstens abschnittsweise ummantelnden zweiten Kühlvolumen von vorgebbarer zweiter Kühltemperatur zum Kühlen des entnommenen Fluids unter die Fluid-Entnahmetemperatur.

Ferner besteht die Erfindung in einem Verfahren zum Abfüllen und Abkühlen einer bei einer vorgebbaren Lagerungs-Temperatur einzulagernden Fluid-Probe eines an einer Fluid-Entnahmestelle entnommenen Fluids, das eine momentane Entnahme-Temperatur aufweist, die größer als die Lagerungs-Temperatur ist, mittels eines Probenehmers, der umfaßt:
- eine Gefäßanordnung von vorgebbarem Lumen mit
   -- einer über das Lumen räumlich gemittelten Gefäßanordnungs-Innentemperatur,
   -- einem rohrförmigen Entnahmegefäß zum Führen des entnommenen Fluids und
   -- einem Speichergefäß zum Speichern der Fluid-Probe, sowie
- eine mit der Gefäßanordnung thermisch gekoppelte Kühlanordnung zum Einstellen der Gefäßanordnungs-Innentemperatur mit
   -- einem wenigstens das Speichergefäß umhüllenden ersten Kühlvolumen von vorgebbarer erster Kühltemperatur zum Kühlen der Fluid-Probe auf die Lagerungs-Temperatur,
- wobei die Gefäßanordnungs-Innentemperatur vor dem Abfüllen eine Anfangs-Gefäßanordnungs-Innentemperatur aufweist, die niedriger als die Entnahme-Temperatur ist und,
- wobei die erste Kühltemperatur nach dem Einlagern der Fluid-Probe auf die Lagerungs-Temperatur eingestellt ist, welches Verfahren folgende Schritte umfaßt:
- die Gefäßanordnungs-Innentemperatur wird mittels der Kühlanordnung auf eine Abkühlungs-Gefäßanordnungs-Innentemperatur abgesenkt, die niedriger als die Anfangs-Gefäßanordnungs-Innentemperatur ist,
- das entnommene Fluid wird durch das Entnahmegefäß hindurchströmen gelassen und
- ein als Fluid-Probe dienendes Teil-Volumen des entnommenen Fluids wird in das Speichergefäß einströmen gelassen.

Nach einer ersten Ausgestaltung des Probenehmers der Erfindung ist das zweite Kühlvolumen vom ersten Kühlvolumen wenigstens teilweise umhüllt.

Nach einer zweiten Ausgestaltung des Probenehmers der Erfindung umfaßt die Gefäßanordnung ein auslaßseits temporär verschließbaren Dosiergefäß zum Abdosieren der Fluid-Probe vom entnommenen Fluid.

Nach einer dritten Ausgestaltung des Probenehmers der Erfindung umfaßt die Gefäßanordnung ein Verteilergefäß zum Einfüllen der Fluid-Probe in das Speichergefäß.

Nach einer vierten Ausgestaltung des Probenehmers der Erfindung umfaßt die Kühlanordnung ein am Entnahmegefäß angeordnetes erstes Kühlelement zum Einstellen einer Entnahmegefäß-Innentemperatur.

Nach einer fünften Ausgestaltung des Probenehmers der Erfindung umfaßt die Kühlanordnung ein am Dosiergefäß angeordnetes zweites Kühlelement zum Einstellen einer Dosiergefäß-Innentemperatur.

Nach einer sechsten Ausgestaltung des Probenehmers der Erfindung umfaßt die Kühlanordnung ein am Verteilergefäß angeordnetes drittes Kühlelement zum Einstellen einer Speichergefäß-Innentemperatur.

Nach einer siebenten Ausgestaltung des Probenehmers der Erfindung umfaßt die Kühlanordnung ein am Speichergefäß angeordnetes viertes Kühlelement zum Einstellen der Speichergefäß-Innentemperatur.

Nach einer achten Ausgestaltung des Probenehmers der Erfindung dient ein Durchlaufkühler als erstes Kühlelement.

Nach einer neunten Ausgestaltung des Probenehmers der Erfindung ist das Dosiergefäß innerhalb des ersten Kühlvolumens angeordnet.

Nach einer zehnten Ausgestaltung des Probenehmers der Erfindung ist das Entnahmegefäß vom ersten Kühlvolumen teilweise umhüllt.

Nach einer elften Ausgestaltung des Probenehmers der Erfindung ist das Fluid ein Trinkwasser oder ein Abwasser.

Nach einer ersten Ausgestaltung des Verfahrens der Erfindung wird die Gefäßanordnungs-Innentemperatur dadurch auf die Abkühlungs-Gefäßanordnungs-Innentemperatur eingestellt, daß die erste Kühltemperatur temporär auf eine erste Abkühlungs-Temperatur abgesenkt wird, die niedriger als die Lagerungs-Temperatur ist.

Nach einer zweiten Ausgestaltung des Verfahrens der Erfindung wird eine Kühlanordnung mit einem die Gefäßanordnung wenigstens abschnittsweise umgebenden zweiten Kühlvolumen von vorgebbarer zweiter Kühltemperatur zum Kühlen des entnommenen Fluids unter die Entnahme-Temperatur verwendet und wird die Abkühlungs-Gefäßanordnungs-Innentemperatur vor dem Einströmenlassen von Fluid in das Speichergefäß durch ein temporäres Einstellen der zweiten Kühltemperatur auf eine zweite Abkühlungs-Temperatur, die niedriger als die Lagerungs-Temperatur ist, abgesenkt.

Nach einer dritten Ausgestaltung des Verfahrens der Erfindung wird die Fluid-Probe von einem wässrigen Fluid entnommen.

Ein Grundgedanke des Verfahrens der Erfindung besteht darin, die Aktivität des entnommenen Fluids und damit auch die der Fluid-Probe bereits während des Abfüllens abzusenken und in möglichst kurzer Zeit ein gefordertes Minimum der Aktivität zu erreichen. Dies wird bei der Erfindung dadurch erreicht, daß ein fluidführendes Lumen der Gefäßanordnung vor dem Abfüllen mittels einer entsprechenden Kühlanordnung abgekühlt wird. Dieses abkühlbare Lumen kann sich dabei über ein Gesamt-Innenvolumen der Gefäßanordnung erstrecken, wodurch die Abkühlung des Fluids unmittelbar mit dessen Einströmen in die Gefäßanordnung beginnt, oder nur einen Teil des Gesamt-Innenvolumens umfassen.

Ein Vorteil des Verfahrens der Erfindung ist, daß die Aktivität der Fluid-Probe, insb. auch bei höheren Fluid-Temperaturen an der Fluid-Entnahmestelle, sehr rasch absenkbar ist. Ein weiterer Vorteil des Verfahrens besteht darin, daß es auch bei bereits bestehenden Gefäßanordnungen anwendbar ist.

Die Erfindung und weitere Vorteile werden nachfolgend anhand der Figuren der Zeichnung näher erläutert, in der Ausführungsbeispiele der Erfindung dargestellt sind.
- Fig. 1: zeigt schematisch ein erstes Ausführungsbeispiel für einen Probenehmer,
- Fig. 2: zeigt schematisch ein zweites Ausführungsbeispiel für einen Probenehmer und
- Fig. 3: zeigt schematisch Temperatur-Zeitverläufe im Probenehmer gemäß Fig. 1 oder 2, die mittels des Verfahrens der Erfindung eingestellt werden.

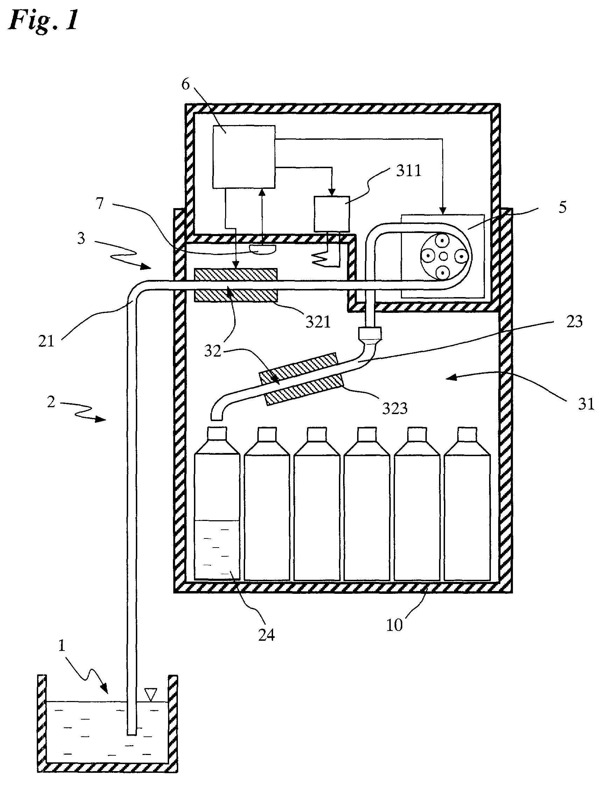

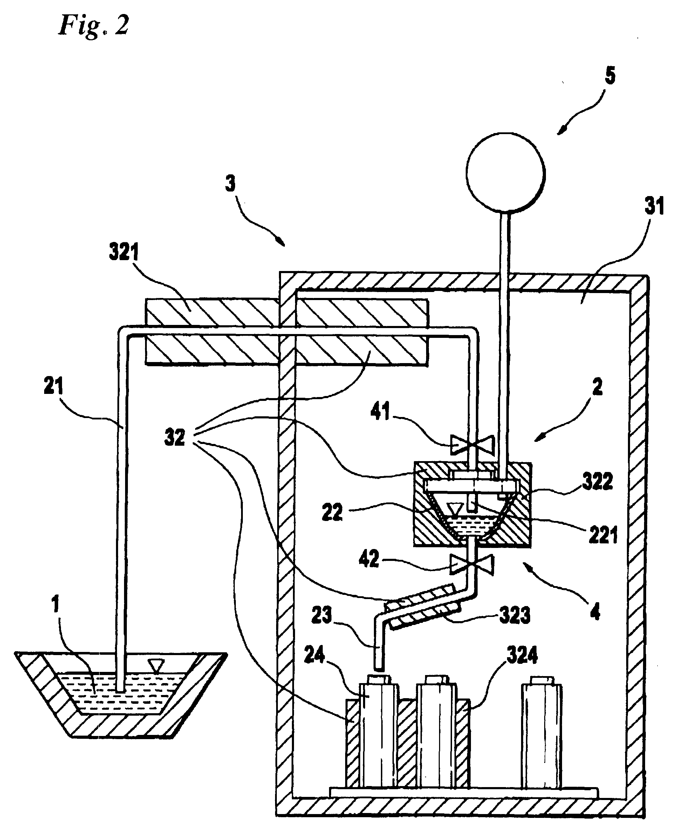

In den Fig. 1 und 2 ist jeweils ein Probenehmer zum Entnehmen eines, insb. wäßriges, Fluids von einer Fluid-Entnahmestelle 1 und zum Abdosieren, Abfüllen und Speichern einer Fluid-Probe des Fluids schematisch dargestellt. Dieser Probenehmer umfaßt dazu eine Gefäßanordnung 2 von vorgebbarem Lumen, die dem Führen und Halten des entnommenen Fluids bzw. der Fluid-Probe dient und die zumindest teilweise in einem, insb. einzigen, Probenehmer-Gehäuse 10 angeordnet ist.

Der Probenehmer kann sowohl ein ortsfester als auch ein mobiler Probenehmer sein.

Die Gefäßanordnung 2 umfaßt mindestens ein Entnahmegefäß 21 zum Entnehmen und Führen des Fluids sowie, zumindest im Betrieb, ein Speichergefäß 24 zum Speichern der einzulagernden Fluid-Probe.

Das Entnahmegefäß 21 ist rohrförmig ausgeführt und weist ein einlaßseitiges erstes und ein auslaßseitiges zweites Ende auf. Bevorzugt ist das Entnahmegefäß 21, zumindest abschnittsweise, als flexibler Schlauch, insb. von elastischem Material, ausgebildet. Als Material können die für derartige Entnahmegefäße üblichen Materialien, wie z.B. Polyethylen und ggf. auch Glas, verwendet werden.

Nach einer vorteilhaften Weiterbildung der Erfindung umfaßt die Gefäßanordnung 2 des Probenehmers gemäß den Fig. 1 und 2 ferner ein Verteilergefäß 23 zum Einleiten der abdosierten Fluid-Probe in das Speichergefäß 24.

Das Verteilergefäß 23 ist wie das Entnahmegefäß 21 ebenfalls rohrförmig, insb. abschnittsweise elastisch verformbar, ausgeführt. Es ist so geformt und dimensioniert, daß ein durch dessen auslaßseitiges Ende strömendes Fluid durch eine Einlaß-Öffnung des Speichergefäßes 24 in dieses einleitbar ist.

Für den Fall, daß das Verteilergefäß 23, wie in den Fig. 1 und 2 dargestellt, nur ein einziges auslaßseitiges Ende aufweist jedoch mittels des Probenehmers nacheinander mehrere Fluid-Proben abgefüllt werden sollen, ist das Verteilergefäß 23 bevorzugt verschwenkbar so ausgeführt, daß ein durch das zweite Ende strömendes Fluid in weitere, vom Speichergefäß 24 beabstandete Speichergefäße einleitbar ist. Das Verteilergefäß 23 kann aber auch mehrere auslaßseitige Enden aufweisen und so ausgeführt sein, daß bei mehreren zu befüllenden Speichergefäßen jedem eines der auslaßseitigen Enden so zugeordnet ist, daß das durch die Enden strömende Fluid in das jeweilige Speichergefäß einleitbar ist.

Außerdem kann das Verteilergefäß 23, für den Fall, daß es direkt an das Entnahmegefäß 21 angeschlossen ist, z.B. auch mittels eines endseitigen Abschnittes des Entnahmegefäßes 21 realisiert sein, der in geeigneter Weise zum jeweils zu befüllenden Speichergefäß verschwenkbar ist, wie dies z.B. in der US-A 44 15 011 vorgeschlagen ist.

Nach einer weiteren vorteilhaften Weiterbildung der Erfindung umfaßt die Gefäßanordnung 2 gemäß Fig. 2 ein Dosiergefäß 22 zum Aufnehmen eines Fluid-Teilvolumens vom einströmen gelassenen Fluid und zum Abdosieren der Fluid-Probe vom Fluid-Teilvolumen. Dazu ist das Entnahmegefäß 21 mit seinem zweiten Ende mit einer ersten Ein/Auslaß-Öffnung des Dosiergefäßes 22 verbunden, die sich bevorzugt an einem höchsten Punkt des Dosiergefäßes 22 bzw. in dessen Nähe befindet; falls erforderlich, kann die Ein/Auslaß-Öffnung z.B. auch an einem tiefer liegenden Punkt des Dosiergefäßes 22 angeordnet sein.

Das Dosiergefäß 22 weist, wie bei derartigen Dosiergefäßen üblich, ein von der Ein/Auslaß-Öffnung ausgehendes und mit einem Ende in dessen Lumen hineinragendes rohrförmiges Ein/Auslaß-Stück 221 von vorgebbarer Länge auf. Für den Fall, daß die vorgebbare Länge des Ein/Auslaß-Stücks im Betrieb des Probenehmers nicht variierbar ist, ist das Ein/Auslaß-Stück 221 in vorteilhafter Weise durch einen auslaßseitigen Abschnitt des Entnahmegefäßes 21, der von oben senkrecht in das Lumen des Dosiergefäßes 22 hineinragt, realisiert.

Das Dosiergefäß 22 besteht bevorzugt aus Glas; es kann aber auch aus jedem anderen, für derartige Dosiergefäße üblichem Material, wie z.B. Polyethylen, bestehen.

Bei dieser Weiterbildung der Erfindung gemäß Fig. 2 ist das Verteilergefäß 23 mit einem einlaßseitigen ersten Ende an eine temporär verschließbare Auslaß-Öffnung des Dosiergefäßes 22 angeschlossen. Die Auslaß-Öffnung befindet sich bevorzugt an einem tiefsten Punkt des Dosiergefäßes 22; sie kann, falls erforderlich, auch an einem anderen Punkt des Dosiergefäßes 22 angeordnet sein.

Gemäß der Fig. 2 umfaßt der Probenehmer bei dieser Weiterbildung der Erfindung des weiteren eine Absperr-Anordnung 4, die dem temporären und abschnittsweisen druckdichten Verschließen der Gefäßanordnung 2, insb. des Dosiergefäßes 22, dient.

Zum temporären Verschließen der Ein/Auslaß-Öffnung des Dosiergefäßes 22 ist an dieser bzw. am Entnahmegefäß 21 ein erstes Absperr-Element 41 der Absperr-Anordnung 4 angebracht. Des weiteren ist zum temporären Verschließen der Auslaß-Öffnung des Dosiergefäßes 22 an dieser bzw. am Verteilergefäß 23 ein zweites Absperr-Element 42 der Absperr-Anordnung 4 angebracht. Als Absperr-Elemente 41, 42 können üblicherweise verwendete manuell, elektromechanisch oder pneumatisch angetriebenen Ventile bzw. Schieber dienen, vgl. die US-A 37 95 347 oder die US-A 38 80 011. Für den Fall, daß ein mindestens abschnittsweise elastisches Verteilergefäß 23 verwendet wird, kann das Absperr-Element auch als eine Schlauchklemme ausgebildet sein, vgl. die US-A 40 77 263.

Bei dem mittels des Probenehmers zu entnehmenden Fluid handelt es sich um ein Fluid, insb. Trinkwasser oder Abwasser, das von der der Fluid-Entnahmestelle 1 entfernt auf seine chemischen und/oder biologischen Eigenschaften, insb. die mitgeführten Substanzen oder Bakterien, zu untersuchen ist. Derartige Fluide weisen eine temperaturabhängige Aktivität auf, die nach der Entnahme eine Veränderung dieser zu untersuchenden chemisch-biologischen Eigenschaften bewirken kann. Die Aktivität ist dabei umso größer, je höher ein momentaner Temperaturwert des Fluids ist. Dementsprechend ist auch die Aktivität der Fluid-Probe umso höher, je höher deren Probentemperatur T_{FP} eingestellt ist. Daher wird das bei einer momentanen Entnahme-Temperatur T₁ entnommenen Fluid nach dessen Entnahme bei einer vorgebbaren niedrigen, insb. stationären, Lagerungs-Temperatur T_{L} eingelagert. Diese Lagerungs-Temperatur T_{L} ist so bemessen, daß die resultierende Aktivität soweit verringert ist, daß sie die Fluid-Probe nicht unzulässig verändert.

Üblicherweise erfolgt die Entnahme des Fluids zumeist bei einer relativ hohen Entnahme-Temperatur T₁, von z.B. 288 K (Kelvin), bei der das Fluid eine entsprechend große Aktivität aufweisen kann. Dies wiederum erfordert ein möglichst schnelles Abkühlen der Fluid-Probe, d.h. eine zwischen dem Beginn der Entnahme und dem Erreichen der Lagerungs-Temperatur T_{L} durch die Fluid-Probe andauernde Abkühlungs-Phase Δta ist zu minimieren.

Der Probenehmer umfaßt daher des weiteren eine mit der Gefäßanordnung 2 thermisch gekoppelte Kühlanordnung 3 zum Kühlen des entnommenen Fluids. Thermisch gekoppelt heißt, daß zwischen der Kühlanordnung 3 und der Gefäßanordnung 2, insb. einem Lumen der Gefäßanordnung 2, temporär vorhandene Temperaturunterschiede, insb. mit einer geringen Verzögerung und nahezu vollständig, ausgeglichen werden können.

Die Kühlanordnung 3 umschließt ein im Betrieb des Probenehmers wenigstens das Speichergefäß 24 umhüllendes erstes Kühlvolumen von vorgebbarer räumlich gemitteiter erster Kühltemperatur T₃₁. Das Kühlvolumen 31 ist, wie in den Fig. 1 und 2 dargestellt, durch das Lumen eines Kühlraums gebildet. Dieser ist bevorzugt direkt im Probenehmer-Gehäuse 10 untergebracht kann dementsprechend z.B. als ein mobiler Kühlbehälter oder als eine ortsfeste Kühlkammer ausgeführt sein.

Das Kühlvolumen 31 ist durch eine, insb. doppelwandige, äußere Umhüllung umschlossen, und so nach außen hin abgegrenzt. Die Umhüllung dient hierbei sowohl der Wärmeisolierung des Kühlvolumens 31 und als Bestandteil des des Probenehmer-Gehäuses 10 gleichzeitig auch als Stützund Haltekonstruktion für die Gefäßanordnung 2. Sie besteht daher bevorzugt zum einen aus wärmeisolierendem Material, wie z.B. PUR-Schaum, und andererseits aus mechanisch festem Konstruktionsmaterial, wie z.B. Edelstahl oder Polyethylen.

Wie in Fig. 2 dargestellt kann das Kühlvolumen 31 so ausgeführt sein, daß es sich zusätzlich auch über das ggf. vorhandene Dosiergefäß 22 und/oder auch über einen Teil des Entnahmegefäßes 21 erstreckt. Ferner ist es bevorzugt so ausgeführt, daß darin neben dem Speichergefäß 24 gleichzeitig noch weitere Speichergefäße untergebracht werden können.

Zu Beginn des Entnahmebetriebes des Probenehmers wird das erste Ende des Entnahmegefäßes 21, z.B. durch Eintauchen, so mit der Fluid-Entnahmestelle 1 verbunden, daß es, wie in den Fig. 1 und 2 dargestellt, mit dieser kommuniziert. Die Fluid-Entnahmestelle 1 kann sich dabei in jedem geeigneten Teil-Volumen des zu entnehmenden Fluids befinden.

In das mit der Fluid-Entnahmestelle 1 kommunizierende Entnahmegefäß 21 wird daraufhin das Fluid zu einem ersten Zeitpunkt t₁ einströmen gelassen und so darin weitergeleitet, daß es, für den Fall, daß die Gefäßanordnung 2 das an das Entnahmegefäß 21 angeschlossene Dosiergefäß 22 umfaßt, letzteres zu einem zweiten Zeitpunkt t₂ erreicht.

Wie in den Fig. 1 und 2 schematisch dargestellt, erfolgt die Entnahme dadurch, daß das Entnahmegefäß 21 in das, z.B. in einem offenen Gerinne oder Becken geführte, Fluid eingetaucht und entgegen der Schwerkraft abgezogen wird; es kann aber auch von einer geeigneten Fluid-Entnahmestelle 1 aus in Richtung der Schwerkraft und/oder aus einer Rohrleitung abgezogen werden.

Zum Abziehen des Fluids von der Fluid-Entnahmestelle 1 weisen Probenehmer der beschriebenen Art üblicherweise eine an die Gefäßanordnung 2 angeschlossene Druckquelle 5 auf, die dem Erzeugen eines statischen Drucks von vorgebbarer Größe in einem Lumen der Gefäßanordnung 2, insb. im Lumen des Entnahmegefäßes 21 und ggf. des Dosiergefäßes 22, dient, vgl. auch die US-A 37 95 347, US-A 38 80 011, US-A 40 77 263 oder US-A 55 87 926. Demgemäß können als Druckquelle 5 z.B. Vakuumpumpen, insb. Membranpumpen oder Kolbenpumpen, bzw. auch Verdrängerpumpen, insb. Peristaltikpumpen, verwendet werden. Da es bei Gefäßanordnungen der beschriebenen Art in Verbindung mit einer als Druckquelle 5 dienenden Vakuumpumpe praktisch keine mechanisch bewegten Bauteile gibt, die direkt mit dem Fluid in Berührung stehen, sind solche Gefäßanordnungen üblicherweise verschleißarm und somit von geringem Wartungsaufwand. Demgegenüber sind solche Gefäßanordnungen mit einer als Druckquelle 5 dienenden Verdrängerpumpe zwar mechanisch höher belastet und somit von höherem Wartungsaufwand, jedoch ist eine derartige Gefäßanordnung einfacher und somit kostengünstiger aufgebaut.

Zum Entnehmen des Fluids wird im Lumen zwischen einlaßseitigem und auslaßseitigem Ende des mit der Fluid-Entnahmestelle 1 kommunizierende Entnahmegefäßes 21 eine erste Druckdifferenz erzeugt, die das Einströmen des Fluids in das Entnahmegefäß 21 bewirkt.

Dazu wird beim Probenehmer gemäß dem Ausführungsbeispiel von der Fig. 1 mittels der Druckquelle 5 das Entnahmegefäß 21 abschnittsweise in fluidfördernde Verdrängerbewegungen versetzt. Das Abdosieren der Fluid-Probe erfolgt nunmehr dadurch, daß über ein während dieses Vorgangs im Entnahmegefäß 21 verbleibendendem Totvolumen hinaus ein als Fluid-Probe dienendes Teilvolumen gefördert und direkt zum Speichergefäß 24 weitergeleitet wird, wie dies z.B. in der US-A 46 60 607 gezeigt ist.

Demgenüber wird beim Probenehmer gemäß dem Ausführungsbeispiel von Fig. 2 bei geöffneter Ein/Auslaß-Öffnung und bei geschlossener Auslaß-Öffnung im Lumen des Dosiergefäßes 22 mittels der Druckquelle 5 der statische Druck zunächst mindestens soweit reduziert, daß das daraufhin in das Entnahmegefäß 21 entgegen der Schwerkraft einströmende Fluid mindestens ein höchstes Niveau des Entnahmegefäßes 21 erreicht, vgl. auch die US-A 40 77 263. Daraufhin wird das Fluid solange in das Dosiergefäß 22 einströmen gelassen, bis dieses mit dem Fluid-Teilvolumen befüllt ist. Dieses ist üblicherweise so bemessen, daß ein festlegbarer erster Füllstand im Dosiergefäß 22 erreicht wird, der sich oberhalb des Endes des Ein/Auslaß-Stückes 221 befindet. Nachdem das Fluid-Teilvolumen in das Dosiergefäß 22 eingefüllt ist, wird im zwischen dem Ende des Ein/Auslaß-Stücks 221 und dem ersten Ende des Entnahmegefäßes 21 befindlichen Lumen der Gefäßanordnung 2 eine zweite Druckdifferenz derart erzeugt, daß ein Teil vom Fluid-Teilvolumen durch das Ein/Auslaß-Stück 221 hindurch wieder zurück in das Entnahmegefäß 21 und von dort weiter zur Fluid-Entnahmestelle 1 strömt. Das Zurückströmen des Fluids erfolgt genau so lange, bis ein zweiter Füllstand das Ende des Ein/Auslaß-Stück 221 erreicht und sich somit im Dosiergefäß 22 nur noch ein als Fluid-Probe dienendes Fluid-Restvolumen befindet. Die zweite Druckdifferenz kann z.B. dadurch erzeugt werden, daß mittels der Druckquelle 5 der statische Druck im Dosiergefäß 22 mindestens soweit erhöht wird, daß das daraufhin in das Ein/Auslaß-Stück 221 und in das Entnahmegefäß 21 entgegen der Schwerkraft einströmende Fluid mindestens das höchste Niveau des Entnahmegefäßes 21 erreicht. Falls sich, wie in der Fig. 2 dargestellt, das Ende des Ein/Auslaß-Stücks 221 auf einem höheren Niveau als ein Fluid-Pegel an der Fluid-Entnahmestelle 1 befindet und damit zwischen beiden eine Höhendifferenz besteht, wird diese Druckdifferenz bevorzugt dadurch erzeugt, daß zwischen der Fluid-Entnahmestelle 1 und dem Dosiergefäß 22 ein Ausgleich von deren statischen Drücken bewirkt wird. Dieser Ausgleich kann bei einer zur Atmosphäre hin geöffneten Fluid-Entnahmestelle 1 z.B. durch einfaches Belüften des Dosiergefäßes 22 erfolgen. Die resultierende Druckdifferenz wird dann im wesentlichen durch die zwischen dem Ende des Ein/Auslaß-Stücks 221 und dem Fluid-Pegel bestehende Höhendifferenz bestimmt. Nachdem das Dosiergefäß 22 nunmehr praktisch nur noch die Fluid-Probe faßt, wird zu einem dritten Zeitpunkt t₃ die Auslaß-Öffnung geöffnet und das Fluid-Restvolumen mittels des Verteilergefäßes 23 in das Speichergefäß 24 abgefüllt.

Die im Speichergefäß 24 befindliche Fluid-Probe wird nach dem Erreichen der im wesentlichen stationären Lagerungs-Temperatur T_{L}, mit einem Temperaturwert, von z.B. 277 K (Kelvin), zu einem Zeitpunkt t₄ eingelagert. Die Lagerungs-Temperatur T_{L} ist dabei höchstens gleich einer zulässigen höchsten Lagerungs-Temperatur des Fluids jedoch mindestens gleich einer zulässigen tiefsten Lagerungs-Temperatur des Fluids. Für den Fall, daß verschiedene Fluide im Kühlvolumen 31 untergebracht sind, ist die zulässige höchste Lagerungs-Temperatur gleich einer niedrigsten der jeweils zulässigen höchsten bzw. ist die zulässige tiefste Lagerungs-Temperatur gleich einer höchsten der jeweils zulässigen tiefsten Lagerungs-Temperatur der Fluide.

In entsprechender Weise ist die Kühltemperatur T₃₁ im Betrieb des Kühlvolumens 31, insb. bei darin befindlichen Fluid-Proben, stets höchstens gleich der zulässigen höchsten Lagerungs-Temperatur eingestellt und somit praktisch auch stets niedriger als eine Umgebungs-Temperatur außerhalb des Kühlvolumens 31. Die Kühltemperatur T₃₁ wird, wie bei derartigen Kühlräumen üblich, mittels einer mit dem Kühlvolumen 31 thermisch gekoppelten aktiven Wärmesenke 311, z.B. mittels einer Wärmepumpe oder eines Peltier-Element, eingestellt. Zum Einstellen der Kühltemperatur T₃₁ können dem Fachmann bekannte Temperatursteuerungs- oder Temperaturregelungsverfahren zum Einsatz gelangen.

Zur Implementierung der für den Betrieb des Probenehmers, insb. für die Entnahme des Fluids und die Abkühlung der Fluid-Probe, erforderlichen Steuer-und/oder Regelverfahren umfaßt der Probenehmer eine entsprechende, ebenfalls im Probenehmer-Gehäuse 10 untergebrachte Betriebsschaltung 6. Die Betriebsschaltung 6 wird bevorzugt von einer externen Energieversorgung, insb. mit einer Wechselspannung im Bereich von 230 V, versorgt. Ferner kann die Betriebsschaltung 6, insb. für den Fall, daß der Probenehmer wie bereits erwähnt transportabel ausgeführt ist, z.B. auch von einem Bordnetz eines entsprechenden Transportfahrzeugs, von einem ebenfalls im Probenehmer-Gehäuse angeordneten Akkumulator und/oder aber auch mittels Solarzellenanordnung energieversorgt werden. Außerdem kann die Betriebsschaltung 6 auch entsprechende Ein/Ausgabe-Schnittstellen, insb. auch für eine manuelle Bedienung, umfassen.

Nachfolgend sollen nunmehr weitere Schritte des Verfahrens erläutert werden. Im Betrieb des Probenehmers liegt zu jedem Zeitpunkt im Lumen der Gefäßanordnung 2, insb. an und in deren Wandungen, jeweils eine momentane Innentemperatur-Verteilung mit einer über diese räumlich gemittelten Gefäßanordnungs-Innentemperatur T₂ vor. Die Höhe der Gefäßanordnungs-Innentemperatur T₂ ist aufgrund von Wärmeübergängen bzw. Wärmeleitung sowie aufgrund von Konvektion in der Gefäßanordnung 2 auch von der Höhe der Kühltemperatur T₃₁ abhängig.

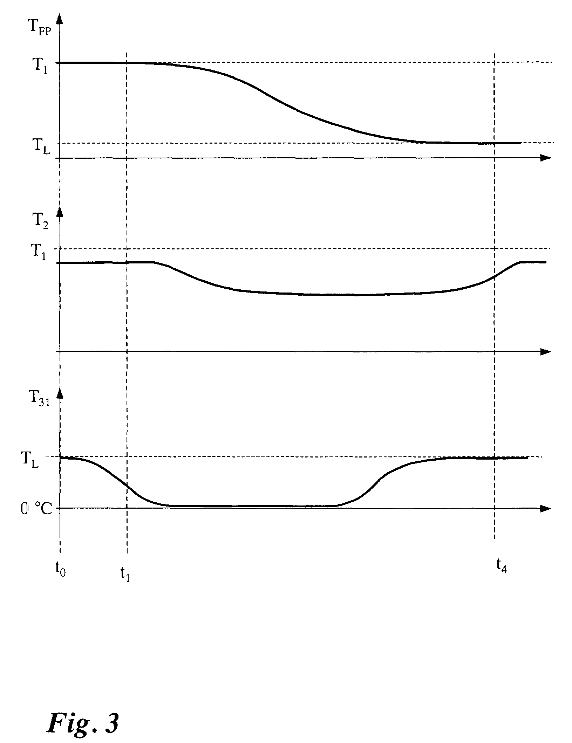

Zu einem Anfangs-Zeitpunkt t₀ vor der Entnahme des Fluids ist die Kühltemperatur T₃₁, wie in Fig. 3 dargestellt, auf die Lagerungs-Temperatur T_{L} des Fluids eingestellt. Somit ist zum Anfangs-Zeitpunkt t₀ eine momentane Anfangs-Innentemperatur-Verteilung mit einer entsprechenden mittleren Anfangs-Gefäßanordnungs-Innentemperatur ausgebildet, die niedriger als die Entnahme-Temperatur T₁ ist.

Da das Kühlvolumen 31 gemäß der Fig. 2, 1 die Gefäßanordnung 2 nur teilweise umschließt, ist deren Innentemperatur-Verteilung, insb. bei einem außerhalb des Kühlvolumens 31 nicht- oder nur teilweise wärmeisoliertem Entnahmegefäß 21 bzw. Dosiergefäß 22, durch eine äußere Umgebungstemperatur-Verteilung entsprechend mitbeeinflußt.

Je weiter sich das Kühlvolumen 31 über die Gefäßanordnung 2 erstreckt und/oder je niedriger die Kühltemperatur T₃₁ über die Abkühlungs-Phase Δta zeitlich gemittelt eingestellt ist, desto kleiner ist ein entsprechendes Temperatur-Verhältnis T₂, _{Δta}/T_{L} von über die Abkühlungs-Phase Δta zeitlich gemittelter Gefäßanordnungs-Innentemperatur T_{2, Δta} zur Lagerungs-Temperatur T_{L}. Die Abkühlungs-Phase Δta ergibt sich dabei aus der zeitlichen Differenz t₄-t₁, die vom Beginn der Entnahme zum Zeitpunkt t₁ bis zum Zeitpunkt t₄ andauert, bei dem die Fluid-Probe die Lagerungs-Temperatur T_{L} erreicht hat.

Je kleiner dieses Temperatur-Verhältnis T₂, _{Δta}/T_{L} ist, desto größer ist im jeweiligen Probenehmer eine ebenfalls über der Abkühlungs-Phase Δta gemittelte Abkühlungs-Geschwindigkeit des in der Gefäßanordnung 2 befindlichen Fluids. Eine Erhöhung der Abkühlungs-Geschwindigkeit verkürzt gleichzeitig die Zeit bis die Fluid-Probe die Lagerungs-Temperatur T_{L} erreicht hat und damit die Abkühlungs-Phase Δta.

Gemäß dem Verfahren der Erfindung wird daher vor der Entnahme des Fluids die Gefäßanordnungs-Innentemperatur von der Anfangs-Gefäßanordnungs-Innentemperatur, z.B. in der Größenordnung von 1 K bis 5 K, auf eine über die Gefäßanordnung 2 gemittelte, insb. zum Zeitpunkt t₁, niedrigere Abkühlungs-Gefäßanordnungs-Innentemperatur abgesenkt, vgl. Fig. 3. Falls erforderlich kann die Anfangs-Gefäßanordnungs-Innentemperatur aber auch um mehr als 5 K abgesenkt werden.

Nach einer vorteilhaften Ausgestaltung des Verfahrens der Erfindung wird die Kühltemperatur T₃₁ dazu mittels der aktiven Wärmesenke 311 von der Lagerungs-Temperatur T_{L} temporär auf eine Abkühlungs-Temperatur, von z.B. 273 K (= 0 °C), abgesenkt. Dadurch ändert sich entsprechend auch die gesamte Innentemperatur-Verteilung im Lumen der Gefäßanordnung 2.

Während oder nach dem Absenken der Kühltemperatur T₃₁ wird das Fluid in der beschriebenen Weise in die Gefäßanordnung 2 einströmen gelassen. Das entnommene Fluid wird daraufhin aufgrund der innerhalb der Gefäßanordnung 2 bestehenden niedrigeren Abkühlungs-Gefäßanordnungs-Innentemperatur unter die Entnahme-Temperatur T₁ abgekühlt. Dies erfolgt im wesentlichen durch Wärmeübergang zu bzw. durch Wärmeleitung in der Wandung der Gefäßanordnung 2. Das Abkühlen der Fluid-Probe beginnt somit praktisch mit dem Einströmen des entnommenen Fluids in den vom Kühlvolumen 31 umschlossenen Teil der Gefäßanordnung 2 und erfolgt, insb. im Speichergefäß 24 nunmehr so lange, bis die Fluid-Probe die eingestellte Kühltemperatur T₃₁ angenommen hat. Da die für die Fluid-Probe einzustellende Lagerungs-Temperatur T_{L} jedoch höher als die momentan abgesenkte Kühltemperatur T₃₁ sein soll, wird diese, wie in Fig. 3 gezeigt, nach einer gewissen Zeit wieder, insb. auf den zum Zeitpunkt t₀ eingestellten Wert, angehoben.

Das Absenken und Wiederanheben der Kühltemperatur T₃₁, und damit auch das Abkühlen der Fluid-Probe innerhalb der Gefäßanordnung 2, insb. innerhalb des Speichergefäßes 24, kann geregelt und/oder zeitgesteuert erfolgen. Die entsprechenden Regelungs- bzw. Steuerungsgrößen sind durch entsprechende Kalibriermessungen ermittelbar. Zur Realisierung des erfindungsgemäßen Verfahrens können in der dem Fachmann bekannten Weise z.B. entsprechende Programmcodes dienen, die in einen Mikrocomputer der Betriebsschaltung 6 implementiert sind und ausgeführt werden. Die zur Durchführung des Verfahrens erforderlichen Stellsignale, z.B. für die Druckquelle 5 oder die Wärmesenke 311, werden, wie in der Fig. 1 schematisch dargestellt, von der Betriebsschaltung 6 geliefert und können z.B. von mittels des Mikrocomputers angesteuerten Signal-Ausgangskarten erzeugt werden.

Während der Abkühlungsphase Δta ist die Kühltemperatur T₃₁, falls erforderlich, auch auf eine niedrigere, unterhalb von 273 K liegende Abkühlungs-Temperatur einstellbar. Die Abkühlungs-Temperatur ist jedoch nur in soweit abzusenken, daß weder unzulässig hohe Abkühlungs-Geschwindigkeiten im Fluid erreicht noch bereits im Kühlvolumen 31 eingelagerte Fluid-Proben unzulässig unterkühlt werden. Daher ist das Absenken bzw. das Wiederanheben der Kühltemperatur T₃₁ ggf. dadurch zu modifizieren, daß die Kühltemperatur T₃₁ während der Abkühlungs-Phase Δta auf Temperatur-Zwischenwerte eingestellt wird, die höher als die Abkühlungs-Temperatur sind. Die entsprechenden Temperatur-Zwischenwerte sind in geeigneter Weise ebenfalls durch entsprechende Kalibrierung ermittelbar.

Falls erforderlich, insb. für den Fall, daß die Kühltemperatur T₃₁ geregelt eingestellt werden soll, ist in der dem Fachmann bekannten Weise ein geeignetes, die Kühltemperatur T₃₁ und/oder die Gefäßanordnungs-Innentemperatur repräsentierendes Meßsignal zu erzeugen. Dazu kann z.B. ein innerhalb des Kühlvolumens 31 angeordneter Temperatursensor 7, wie z.B. ein Widerstandsthermometer oder ein Thermoelement, verwendet werden, der, z.B. via Signal-Eingangskarte, mit der Betriebsschaltung 6, insb. mit vorgenanntem Mikrocomputer, verbunden ist, vgl. hierzu auch die US-A 55 87 926.

Nach einer weiteren Ausgestaltung des Probenehmers der Erfindung umfaßt die Kühlanordnung 3 ein die Gefäßanordnung 2 wenigstens teilweise umschließendes zweites Kühlvolumen 32 von vorgebbarer räumlich gemittelter zweiter Kühltemperatur T₃₂, das insb. innerhalb der Wandung der Gefäßanordnung 2 zumindest abschnittsweise und im dadurch ummantelten Lumen der Gefäßanordnung 2 ausgebildet ist.

Das Kühlvolumen 32 dient einerseits der weiteren Ausdehnung des kühlbaren Lumens der Gefäßanordnung 2 in Richtung der Fluid-Entnahmestelle 1. Andererseits dient es der gezielten Einflußnahme auf die Innentemperatur-Verteilung der Gefäßanordnung 2 und damit einem feineren und genaueren Einstellen der Gefäßanordnungs-Innentemperatur T₂ während der Abkühlungs-Phase Δta.

Gemäß der Fig. 1 bzw. 2 wird das Kühlvolumen 32 durch ein erstes Kühlelement 321 am Entnahmegefäß 21 zum Einstellen einer Entnahmegefäß-Innentemperatur, ein zweites Kühlelement 322 am Dosiergefäß 22 zum Einstellen einer Dosiergefäß-Innentemperatur, ein drittes Kühlelement 323 am Verteilergefäß 23 zum Einstellen einer Verteilergefäß-Innentemperatur und/oder ein viertes Kühlelement 324 am Speichergefäß 24 zum Einstellen der Speichergefäß-Innentemperatur jeweils abschnittsweise umschlossen, und damit praktisch in entsprechende Teil-Kühlvolumen aufgeteilt. Wie in Fig. 1 anhand des Kühlelements 321 stellvertretend gezeigt, werden die erforderlichen Stellsignale ebenfalls von der Betriebsschaltung 6 geliefert.

Als Kühlelemente, insb. als Kühlelement 321, können wiederum entsprechende Wärmepumpen dienen. Nach einer bevorzugten Ausgestaltung der Erfindung ist das Kühlelement 321 als Durchlaufkühler ausgelegt. Des weiteren können auch Peltier-Elemente in den Kühlelementen 321, 322, 323, 324 verwendet werden.

Falls erforderlich, kann das Kühlvolumen 32, wie z.B. auch in der Fig. 1 gezeigt, durch nur drei, zwei oder durch ein einziges dieser Kühlelemente 321, 322, 323, 324 gebildet sein, insb. dann, wenn, wie dargestellt, das Kühlvolumen 32 bevorzugt wenigstens teilweise vom Kühlvolumen 31 umschlossen wird.

Vorteilhaft bei dieser Ausgestaltung der Erfindung ist, daß bereits bestehende Probenehmer ohne weiteres mit derartigen Kühlelementen nachrüstbar sind.

Nach einer weiteren Ausgestaltung des Verfahrens der Erfindung wird die Gefäßanordnungs-Innentemperatur T₂ vor der Entnahme des Fluids mittels eines oder mehrerer der Kühlelemente 321, 322, 323 bzw. 324 temporär auf die Abkühlungs-Gefäßanordnungs-Innentemperatur von z.B. ebenfalls 273 K, abgesenkt, so daß die Gefäßanordnungs-Innentemperatur T₂, insb. zum Zeitpunkt t₁, wiederum niedriger als die Anfangs-Gefäßanordnungs-Innentemperatur ist.

Während oder nach dem Absenken der Kühltemperatur T₃₂ wird das Fluid wiederum in die Gefäßanordnung 2 einströmen gelassen und dadurch auf eine Fluid-Temperatur abgekühlt, die unterhalb der Entnahme-Temperatur T₁ liegt. Selbstverständlich kann bei dieser Ausgestaltung der Erfindung auch das zuvor beschriebene Verfahren, bei dem die Kühltemperatur T₃₁ abgesenkt wird, mit zur Anwendung kommen.

Vor dem Beginn des Entnahmebetriebes werden üblicherweise, insb. bei mehreren aufeinanderfolgenden Abfüllvorgängen, fluidische Rückstände aus der Gefäßanordnung 2 entfernt.

Dies erfolgt beim Ausführungsbeispiel gemäß der Fig. 2 in geeigneter Weise z.B. dadurch, daß bei verschlossener Auslaß-Öffnung des Dosiergefäßes 22 und bei gleichzeitig geöffneter Ein/Auslaß-Öffnung mittels der Druckquelle 5 ein statischer Überdruck im Lumen des Dosiergefäßes 22 und des angeschlossenen Entnahmegefäßes 21 erzeugt wird. Aufgrund dieses Überdruckes werden im Entnahmegefäß 21 befindliche Rückstände durch dessen erstes Ende herausdrückt. Falls erforderlich, kann außerdem durch ein Verschließen der Ein/Auslaß-Öffnung bei gleichzeitig geöffneter Auslaß-Öffnung mittels eines im Lumen des Dosiergefäßes 22 erzeugten Überdrucks das Dosiergefäß selbst sowie das angeschlossene Verteilergefäß 23 von fluidischen Rückständen befreit werden; letztere können dann über das zweite Ende des Verteilergefäßes 23 in ein geeignetes Gefäß des Probenehmers oder aus diesem heraus geleitet werden. Beim Ausführungsbeispiel gemäß der Fig. 1 kann das Entfernen fluidischer Rückstände aus der Gefäßanordnung 2 in einfacher Weise dadurch erfolgen, daß lediglich die als Verdrängerpumpe realsisierte Druckquelle 5 revers, also wiederum in der zum Entnahmebetrieb entgegengesetzten Richtung, betrieben wird.

## Patentansprüche

1. Probenehmer zum Abfüllen und Abkühlen einer bei einer vorgebbaren Lagerungs-Temperatur einzulagernden Fluid-Probe eines an einer Fluid-Entnahmestelle (1) entnommenen Fluids, das eine momentane Entnahme-Temperatur aufweist, die größer als die Lagerungs-Temperatur ist, welcher Probenehmer umfaßt:
- eine Gefäßanordnung (2) von vorgebbarem Lumen mit
-- einer über das Lumen räumlich gemittelten Gefäßanordnungs-Innentemperatur
-- einem rohrförmigen Entnahmegefäß (21) zum Führen des entnommenen Fluids und
-- einem Speichergefäß (24) zum Speichern der Fluid-Probe sowie
- eine mit der Gefäßanordnung (2) thermisch gekoppelte Kühlanordnung (3) zum Einstellen der Gefäßanordnungs-Innentemperatur, mit
-- einem wenigstens das Speichergefäß (24) umhüllenden ersten Kühlvolumen (31) von vorgebbarer erster Kühltemperatur zum Kühlen der Fluid-Probe auf die Lagerungs-Temperatur und
-- einem die Gefäßanordnung (2) wenigstens abschnittsweise ummantelnden zweiten Kühlvolumen (32) von vorgebbarer zweiter Kühltemperatur zum Kühlen des entnommenen Fluids unter die Fluid-Entnahmetemperatur.

2. Probenehmer nach Anspruch 1, bei dem das zweite Kühlvolumen (32) vom ersten Kühlvolumen (31) wenigstens teilweise umhüllt ist.

3. Probenehmer nach Anspruch 1 oder 2, bei dem die Gefäßanordnung (2) ein auslaßseits temporär verschließbaren Dosiergefäß (22) zum Abdosieren der Fluid-Probe vom entnommenen Fluid umfaßt.

4. Probenehmer nach einem der Ansprüche 1 bis 4, bei dem die Gefäßanordnung (2) ein Verteilergefäß (23) zum Einfüllen der Fluid-Probe in das Speichergefäß (24) umfaßt.

5. Probenehmer nach einem der Ansprüche 1 bis 4, bei dem die Kühlanordnung (3) ein am Entnahmegefäß (21) angeordnetes erstes Kühlelement (321) zum Einstellen einer Entnahmegefäß-Innentemperatur umfaßt.

6. Probenehmer nach einem der Ansprüche 3 bis 5, bei dem die Kühlanordnung (3) ein am Dosiergefäß (22) angeordnetes zweites Kühlelement (322) zum Einstellen einer Dosiergefäß-Innentemperatur umfaßt.

7. Probenehmer nach einem der Ansprüche 4 bis 6, bei dem die Kühlanordnung (3) ein am Verteilergefäß (23) angeordnetes drittes Kühlelement (323) zum Einstellen einer Speichergefäß-Innentemperatur umfaßt.

8. Probenehmer nach einem der Ansprüche 1 bis 7, bei dem die Kühlanordnung (3) ein am Speichergefäß (24) angeordnetes viertes Kühlelement (324) zum Einstellen der Speichergefäß-Innentemperatur umfaßt.

9. Probenehmer nach Anspruch 5, bei dem ein Durchlaufkühler als erstes Kühlelement (321) dient.

10. Probenehmer nach einem der Ansprüche 3 bis 9, bei dem das Dosiergefäß (22) innerhalb des ersten Kühlvolumens (31) angeordnet ist.

11. Probenehmer nach Anspruch 10, bei dem das Entnahmegefäß (21) vom ersten Kühlvolumen (31) teilweise umhüllt ist.

12. Probenehmer nach einem der Ansprüche 1 bis 11, bei dem das Fluid ein Trinkwasser oder ein Abwasser ist.

13. Verfahren zum Abfüllen und Abkühlen einer bei einer vorgebbaren Lagerungs-Temperatur einzulagernden Fluid-Probe eines an einer Fluid-Entnahmestelle (1) entnommenen Fluids, das eine momentane Entnahme-Temperatur aufweist, die größer als die Lagerungs-Temperatur ist, mittels eines Probenehmers, der umfaßt:
- eine Gefäßanordnung (2) von vorgebbarem Lumen mit
-- einer über das Lumen räumlich gemittelten Gefäßanordnungs-Innentemperatur,
-- einem rohrförmigen Entnahmegefäß (21) zum Führen des entnommenen Fluids und
-- einem Speichergefäß (24) zum Speichern der Fluid-Probe, sowie
- eine mit der Gefäßanordnung (2) thermisch gekoppelte Kühlanordnung (3) zum Einstellen der Gefäßanordnungs-Innentemperatur mit
-- einem wenigstens das Speichergefäß (24) umhüllenden ersten Kühlvolumen (31) von vorgebbarer erster Kühltemperatur zum Kühlen der Fluid-Probe auf die Lagerungs-Temperatur,
- wobei die Gefäßanordnungs-Innentemperatur vor dem Abfüllen eine Anfangs-Gefäßanordnungs-Innentemperatur aufweist, die niedriger als die Entnahme-Temperatur ist und,
- wobei die erste Kühltemperatur nach dem Einlagern der Fluid-Probe auf die Lagerungs-Temperatur eingestellt ist, welches Verfahren folgende Schritte umfaßt:
- die Gefäßanordnungs-Innentemperatur wird mittels der Kühlanordnung (3) auf eine Abkühlungs-Gefäßanordnungs-Innentemperatur abgesenkt, die niedriger als die Anfangs-Gefäßanordnungs-Innentemperatur ist,
- das entnommene Fluid wird durch das Entnahmegefäß (21) hindurchströmen gelassen und
- ein als Fluid-Probe dienendes Teil-Volumen des entnommenen Fluids wird in das Speichergefäß (24) einströmen gelassen.

14. Verfahren nach Anspruch 13, bei dem die Gefäßanordnungs-Innentemperatur dadurch auf die Abkühlungs-Gefäßanordnungs-Innentemperatur eingestellt wird, daß die erste Kühltemperatur temporär auf eine erste Abkühlungs-Temperatur abgesenkt wird, die niedriger als die Lagerungs-Temperatur ist.

15. Verfahren nach Anspruch 13 oder 14,
- bei dem eine Kühlanordnung (3) mit einem die Gefäßanordnung (2) wenigstens abschnittsweise umgebenden zweiten Kühlvolumen (32) von vorgebbarer zweiter Kühltemperatur zum Kühlen des entnommenen Fluids unter die Entnahme-Temperatur verwendet wird und
- bei dem die Abkühlungs-Gefäßanordnungs-Innentemperatur vor dem Einströmenlassen von Fluid in das Speichergefäß (24) durch ein temporäres Einstellen der zweiten Kühltemperatur auf eine zweite Abkühlungs-Temperatur, die niedriger als die Lagerungs-Temperatur ist, abgesenkt wird.

16. Verfahren nach einem der Ansprüche 13 bis 15, bei dem die Fluid-Probe von einem wässrigen Fluid entnommen wird.
